# EUROPEAN PATENT APPLICATION

(11) **EP 3 895 598 A1**
(43) Date of publication of application: **20.10.2021**
(21) Application number: 19897252.3
(22) Date of filing: 01.10.2019
(51) Int. Cl.: A61B 1/253, A61B 1/24, A61B 1/247, A61B 1/06

(54) **DEVICE FOR VIEWING THE INSIDE OF ORAL CAVITIES**

(30) Priority: 12.12.2018 ES 201831902 U
(71) Applicant: Ramon Gariglio, Patricia, Maria, 09004 Burgos (ES)
(72) Inventor: Ramon Gariglio, Patricia, Maria, 09004 Burgos (ES)
(74) Representative: Herrero & Asociados, S.L.
(86) International application number: PCT/ES2019/070654
(87) International publication number: WO 2020/120812

(57) **Abstract**

Oral cavity interior visualisation device for indirect visualisation of the interior of an oral cavity, comprising a handle (1), through the interior of which runs a duct (4) for supplying a compressed air flow, a circular mirror (3), and a support (2) attachable to the handle (1) for suspension of the mirror (3), through the interior of which runs the compressed air flow. The support (2) comprises a semi-circular base (13) located at a distal end of the support (2), and a perimeter groove in the base (13) for housing the mirror (3). The base (13) incorporates a semi-circular air diffuser (14), which extends around the entire perimeter of the base (13), for directing the compressed air circulating inside the support (2) over the surface of the mirror (3) in the form of a continuous laminar flow.

## Description

### Object of the invention

The present invention falls within the technical field of instruments for the medical examination of the mouth, more specifically those equipped with means for preventing the formation of mist and for the removal of water and particles, and relates in particular to a device for visualisation of the interior of oral cavities.

### Background to the invention

Within the technical field of dentistry, instruments, generically known as mirrors, are widely known and used, which allow the visualisation of certain places inside the oral cavity where direct visibility is difficult or impossible. These mirrors also have a number of additional uses, such as being used to reflect a beam of light onto certain parts of the mouth or to retract the tongue, buccal walls or pharynx.

Commercial oral mirrors face three main problems: the mist generated by the patient's mouth breathing, the water and particles that stick to the mirror surface due to the continuous expulsion of this liquid when using the dental drill, and the patient's own saliva.

Also, during examinations and interventions, dentists need to use one hand to hold the mirror, while the other hand is used to handle instruments to polish or make holes and incisions in the teeth, for which they have a built-in system that supplies the aforementioned water, which falls together with dental debris onto the mirror, impeding visibility, making it necessary to stop to clean the mirror.

In the current state of the art, some patent documents are known relating to dentist's mirrors equipped with elements aimed at preventing the accumulation of dirt that prevents correct visualisation. For example, the Spanish utility model with publication number ES1108306U describes a dental mirror incorporating a system for expelling air or gas directly onto the mirror, consisting of a handle and a support with a mirror, with a plurality of holes that project pressurised air directly onto the surface of the mirror, in which said holes are connected through a conduit that runs along the support to a refillable pressurised air cylinder or ampoule housed inside the handle that is operated by means of a manual pushbutton also located on the handle.

The mirror thus described solves the problem, but in a cumbersome and uneconomical way, as it has to be fitted with a compressed air tank, which makes it difficult to manoeuvre.

Similarly, US patent publication number US3986266 describes a dental mirror comprising a handle with a reflecting mirror mounted at one end, an air jet tube attached to the handle and arranged to direct a stream of air to the reflecting face of the mirror, and includes a water supply tube from which water droplets are supplied to the air stream from the air jet tube, so that this water cleans the mirror face of any debris obscuring the dentist's view. This mirror also attempts to solve the technical problem mentioned above by using a mixture of air and water spray to clean the mirror surface.

### Description of the invention

The subject matter of the invention consists of a device for visualisation of the interior of oral cavities, connectable to a compressed air supply pipe, which allows indirect visualisation of the interior of a user's oral cavity.

For this purpose, the device basically comprises a grip handle, a support and a mirror inserted in the support. In the device of the invention, the support and mirror, which are the elements that are usually inserted and come into contact with the oral cavity, are attachable and detachable from the grip handle, in order to be easily interchangeable and thus allow for proper sanitisation without the need to completely disable the device while sanitisation is being carried out.

The device incorporates a diffuser element, configured and sized to project a continuous flow of compressed air onto the surface of the mirror, either from a dental chair of the type normally used by dentists or from an external autonomous system, so that, firstly, an air cushion is created that hinders deposition on the surface of the mirror and prevents the formation of mist and, secondly, it removes water and heavier particles that have been deposited on the mirror by dragging them away.

Additionally, the mirror used in the device incorporates a hydrophobic coating, preferably consisting of a transparent film, in order to prevent the accumulation of liquids, such as saliva or hydration water from the chair.

As mentioned above, it is envisaged that the device can make use of a flow of compressed air from external equipment, independent of that of a dentist's chair, in cases where a suitable connection cannot be made.

The incorporation of additional elements is also envisaged, such as a compressed air flow regulation element, consisting, in a preferred embodiment, of a stopcock that can be manually operated by a user, which can interrupt the flow of compressed air completely if necessary. In addition, light-emitting elements can be incorporated to improve the visualisation of the inside of the oral cavity. In a preferred embodiment, the light-emitting element is an LED-type light, located in the vicinity of the holder and oriented in such a way as to project the emitted light beam towards the inside of the cavity.

The device for visualisation of the interior of oral cavities described above is an effective, simple, economical and hygienic solution to help obtain a clear view of the inside of the mouth, avoiding the problems and inconveniences described above.

### Description of the drawings

In order to complement the description being made and in order to assist in a better understanding of the features of the invention, in accordance with a preferred example of a practical embodiment thereof, a set of drawings is attached hereto as an integral part of said description, in which the following is illustrated for illustrative and nonlimiting purposes:
Figure 1.- Shows a top perspective view of the device for visualisation of the interior of oral cavities, showing its main constituent elements.
Figure 2.- Shows a detail view of the area at the end of the device where the hydrophobic mirror is located.

### Preferred embodiment of the invention

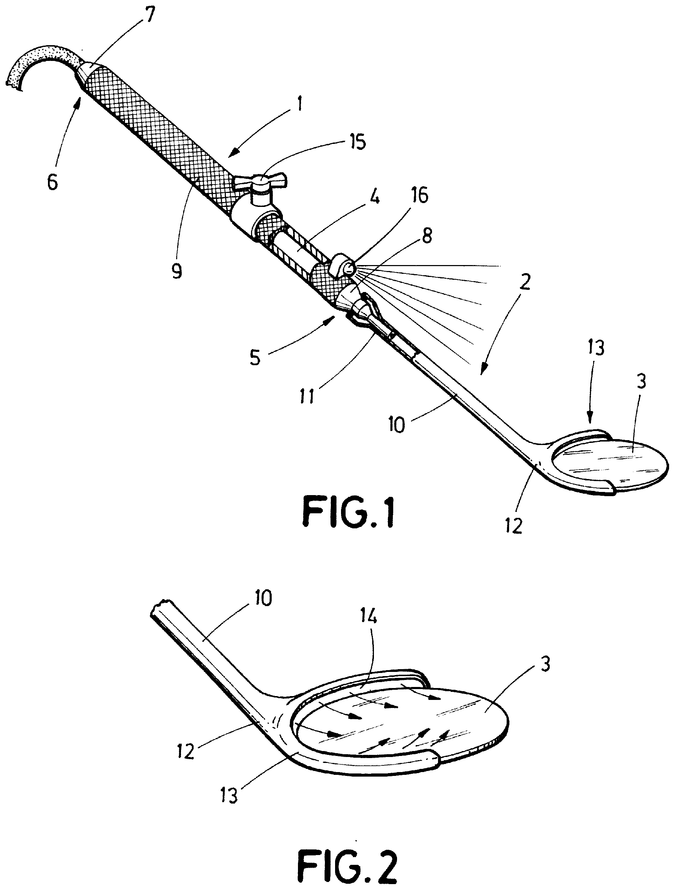

The device for visualisation of the interior of oral cavities, shown schematically in figure 1, consists of a handle (1) connectable to a compressed air pipe, a support (2) attachable to the handle (1) and a circular mirror (3) inserted in a distal end of the support (2), which keeps it suspended.

The handle (1) is in this preferred embodiment a hollow tubular body, through the interior of which runs a duct (4) for supplying a pressurised air flow, and has a front end (5) and a rear end (6). At the rear end (6) there is a connection (7) for connecting the duct (4) either to a compressed air pipe from a dentist's chair or to an external independent source through which the compressed air flow passes. On the other hand, at the front end (5) there is a coupling (8) for connection with the support (2).

In the preferred embodiment described here, the handle (1) incorporates a grooved surface (9) to increase the contact surface, thus facilitating gripping and preventing possible slipping. Likewise, the coupling (8) consists of a conical-concentric reduction of the tubular body that forms the handle (1), as can be seen in the attached figures.

On the other hand, the support (2) comprises a straight and hollow tubular sector (10), through the interior of which the air flow transported by the duct (4) flows, which has a first end where a coupling (11) connectable to the coupling (8) of the handle (1) is located, and a second end where a bend (12) is located, from which a semi-circular base (13) starts, for the attachment of the circular mirror (3).

The base (13) has a lower perimeter groove, not shown in the attached figures, for the insertion and housing of a section of the perimeter edge of the mirror (3), which is thus kept suspended, and a semi-circular air diffuser (14) located above the mirror (3).

The semi-circular diffuser (14) is configured and dimensioned to direct the compressed air that circulates inside the support (2) from the duct (4) towards the surface of the mirror (3) in the form of a continuous laminar flow, which produces, firstly, the dragging and removal of both the liquid and the possible residues accumulated on it, and secondly, it forms a layer of air that prevents the deposition of more debris on the surface of the mirror (3) and prevents the formation of mist and other elements that can fog up the mirror (3) and make it difficult to see properly.

In this preferred embodiment, the semi-circular diffuser (14) consists of a through slot extending around the inner perimeter of the base (13).

Finally, the mirror (3) surface incorporates a hydrophobic coating, consisting of a transparent film, which prevents the accumulation of liquids, such as saliva or hydration water from the chair.

In the preferred embodiment described here, the device incorporates a flow regulator (15), which acts on the duct (4) to manually regulate the flow of compressed air circulating inside it. As can be seen in the attached figures, said flow regulator (15) consists in this embodiment of a key located in the handle (1) of the device.

The device also comprises a light-emitting element (16) which helps to improve the visualisation of the inside of the oral cavity by projecting a light beam. In this preferred embodiment, said light emitter (16) consists of an LED type light, located in the vicinity of the bend (12), oriented in such a way that said light beam is projected towards the oral cavity, without interfering with the surface of the mirror (3), thus avoiding reflections and glare.

## Claims

1. Device for visualisation of the interior of oral cavities for indirect visualisation of the interior of an oral cavity, comprising:
- a handle (1) for gripping, inside which runs a duct (4) for supplying a compressed air flow, connectable to a corresponding compressed air pipe,
- a circular mirror (3), and
- a support (2) attachable to the handle (1) for suspension of the mirror (3), through the interior of which the flow of compressed air transported by the duct (4) flows, the support (2) further comprising:
- a semi-circular base (13) located at a distal end of the support (2), and
- a perimeter groove located in the base (13) to accommodate a section of the perimeter edge of the mirror (3),
being the device **characterised in that** the base (13) incorporates a semi-circular air diffuser (14), extending around the entire perimeter of the base (13), configured and sized to direct the compressed air circulating inside the support (2) over the surface of the mirror (3) in the form of a continuous laminar flow.

2. Device according to claim 1, **characterised in that** the mirror (3) incorporates a hydrophobic coating.

3. Device according to any one of the preceding claims, **characterised in that** the handle (1) is a hollow tubular body comprising:
- an internal cavity for housing the duct (4),
- a front end (5),
- a rear end (6),
- a connection (7) located at the rear end (6) for connection of the duct (4) to the compressed air pipe, and
- a coupling (8) located at the front end (5) for attachment to the bracket (2).

4. Device according to claim 3, **characterised in that** the support (2) comprises:
- a straight and hollow tubular section (10), through which the air flow transported by the duct (4) passes,
- a coupling (11) located at a first end of the tubular section (10), for connection to the coupling (8) of the handle (1), and
- a bend (12) located at a second end of the tubular section (10), from which the semi-circular base (13) starts.

5. Device according to any of the preceding claims, **characterised in that** the handle (1) incorporates a grooved surface (9) for increasing the contact surface.

6. Device according to any of the preceding claims, **characterised in that** it further comprises a flow regulator (15) that can be actuated on the duct (4) for manual regulation of the flow of compressed air circulating inside it.

7. Device according to any one of the preceding claims, **characterised in that** it further comprises a light emitting element (16) for projection of a light beam inside the oral cavity.

8. Display device according to claim 1, **characterised in that** the duct (4) for supplying a compressed air flow is connectable to a corresponding compressed air pipe of a dentist's chair.

9. Display device according to claim 1 **characterised in that** the duct (4) for supplying a compressed air flow is connectable to an external, self-contained compressed air source.
